# EUROPEAN PATENT APPLICATION

(11) **EP 4 350 356 A1**
(43) Date of publication of application: **10.04.2024**
(21) Application number: 22815240.1
(22) Date of filing: 30.05.2022
(51) Int. Cl.: G01N 33/68, A01K 67/027, A61K 49/00, A61K 45/00, A61P 25/16

(54) **PARKINSON'S SYNDROME MARKER AND USE THEREOF**

(30) Priority: 01.06.2021 CN 202110611079
(71) Applicant: Suzhou Biomed Biotechnology Co., Ltd., Suzhou, Jiangsu 215125 (CN)
(72) Inventor: WANG, Changhe, Suzhou, Jiangsu 215125 (CN); KANG, Xinjiang, Suzhou, Jiangsu 215125 (CN); GU, Yuhao, Suzhou, Jiangsu 215125 (CN); XIE, Zhenli, Suzhou, Jiangsu 215125 (CN)
(74) Representative: Lavoix
(86) International application number: PCT/CN2022/096057
(87) International publication number: WO 2022/253195

(57) **Abstract**

Provided is any of the following applications: I) an application of a substance for measuring the content of synaptotagmin-11 in the preparation of a product for diagnosing or assisting the diagnosis of whether a subject suffers from Parkinson's syndrome; and 2) an application of a substance for measuring the content of synaptotagmin-11 in preparing a product for screening or assisting screening of whether a subject suffers from Parkinson's syndrome.

## Description

### TECHNICAL FIELD

The present application relates to the field of biotechnology, in particular to a Parkinson's syndrome marker and use thereof.

### BACKGROUND

Parkinson's disease (PD) is the second largest neurodegenerative disease, the incidence rate of people over 65 years old is more than 1.7%, and the incidence rate of people over 80 years old is 10%. It has been more than 200 years since the disease was first named. There is no specific and efficient detection kit, and no clinical cure.

The main clinical manifestations of PD are movement disorders such as bradykinesia, muscle stiffness, static tremor, and posture and gait instability, accompanied by a series of non-movement disorders such as depression, dementia, sleep disturbance, anosmia, pain, urinary incontinence, constipation, etc. PD is a specific degenerative disease of dopamine (DA) neurons that seriously affects human health and brings a heavy burden to society and family. Non-movement symptoms of PD may appear 10-15 years earlier than movement symptoms, but movement symptoms and DA neuron apoptosis are currently the gold standard for clinical diagnosis of PD. However, clinically, the apoptosis and loss of DA neurons have reached more than 60% when movement symptoms appear. Due to the non-regeneration of neurons, there is currently no effective cure for PD. At present, the main clinical treatment strategies are only limited to alternative therapeutic medicants of DA or palliative treatment plans such as deep brain stimulation. At present, searching for early and efficient diagnostic markers and taking advantage of the 10-year window period of Parkinson's disease to carry out protective or preventive treatment before the death of DA neurons have become important strategies for the prevention and cure of PD, and also the key to achieve the ultimate goal of treating PD before it develops, delaying the onset of PD or preventing PD from developing over the course of a lifetime. Therefore, research on the pathological mechanism of PD and its diagnostic markers has always been one of the most basic, important, and core research contents in this field. According to the MEDLINE database, about 120,000 PD-related literature has been published in the international academic community. Among the projects approved by the US National Health Service over the years (24 years), there are about 22,000 PD studies. However, there are still no relevant reports and clinical applications of PD early diagnostic markers, especially single markers in peripheral blood. The discovery and application of PD early diagnostic markers have been key scientific and medical problems that have not been resolved in this field for more than 200 years.

Parkinson's disease was initially considered to be a sporadic disease. With the emergence of genome-wide association analysis (GWAS) and whole-genome, whole-exome sequencing and other technologies, people have found a large number of PD associated/pathogenic genes, including *PARK1* (SNCA), *PARK2* (Parkin), *PARK6* (PINK1), *PARK7* (DJ-1) and *PARK8* (LRRK2), thus laying the foundation for the genetic mechanism of Parkinson's disease. However, it is currently known that all familial hereditary (gene mutation) PD accounts for only 5% to 10% of all PD cases (data from reference Nat Rev Neurosci 2017,18:251-259; Trend Neurosci 2014,37(6):316-324; Mol Cell Probes 2016,30:386-396; Trend Biochem Sci 2015,40(4):200-210; Neurodegenerative Dis 2007, 4:424-427), gene mutations or genetic factors as diagnostic markers of PD severe restricted. Therefore, the broad consensus formed in this field (such as documented in Nat Rev Dis Primers 2017, 3, 17013; Lancet 2015(386):896-912; Parkinsonism Relat Disord 2016 S1:S106-110) is that genetic factors, especially single genetic factors cannot be used as a broad-spectrum clinical diagnostic marker for the pathological process of PD, and so far no single clinical diagnostic marker that can effectively indicate the pathological process of PD has been found.

### SUMMARY OF THE INVENTION

For this reason, the first purpose of the present application is to provide any of the following use:
1) use of a substance for measuring the content of synaptotagmin-11 in preparing a product for diagnosing or assisting diagnosing of whether a subject to be tested suffers from Parkinson's syndrome;
2) use of a substance for measuring the content of synaptotagmin-11 in preparing a product for screening or assisting screening of whether a subject to be tested suffers from Parkinson's syndrome;
3) use of a substance for measuring the content of synaptotagmin-11 in diagnosing or assisting diagnosing of whether a subject to be tested suffers from Parkinson's syndrome; and
4) use of a substance for measuring the content of synaptotagmin-11 in screening or assisting screening of whether a subject to be tested suffers from Parkinson's syndrome.

Optionally, the synaptotagmin-11 is the synaptotagmin-11 in midbrain tissue or body fluid of the subject to be tested.

Optionally, the body fluid is one or more selected from blood, cerebrospinal fluid and urine.

The second purpose of the present application is to provide any of the following use:
1) use of synaptotagmin-11 as a marker in diagnosing or assisting diagnosing of whether a subject to be tested suffers from Parkinson's syndrome; and
2) use of synaptotagmin-11 as a marker in screening or assisting screening of whether a subject to be tested suffers from Parkinson's syndrome;

optionally, the synaptotagmin-11 is the synaptotagmin-11 in midbrain tissue or body fluid of the subject to be tested;
optionally, the body fluid is one or more selected from blood, cerebrospinal fluid and urine.

The subject to be tested are patients with Parkinson's syndrome, suspected patients with Parkinson's syndrome, high-risk groups of Parkinson's syndrome or immediate relatives of patients with Parkinson's syndrome; patients with Parkinson's syndrome is a sporadic Parkinson's syndrome patient or a familial hereditary Parkinson's syndrome patient.

The third purpose of the present application is to provide a product for diagnosing or assisting diagnosing of whether a subject to be tested suffers from Parkinson's syndrome, wherein the product diagnoses, assisting diagnoses, screens or assisting screens whether a subject to be tested suffers from Parkinson's syndrome based on the expression level of synaptotagmin-11 in biological samples.

Optionally, the product is a kit; optionally, the kit comprises reagents for specifically detecting the expression level of synaptotagmin-11.

Optionally, the reagents comprise an antibody, a protein and/or a polypeptide that specifically bind to the synaptotagmin-11 protein.

Optionally, the antibody, the protein and/or the polypeptide is modified with isotopes, horseradish peroxidase, colloidal gold, fluorescent probes, etc., or is not modified.

The fourth purpose of the present application is to provide a system for diagnosing, assisting diagnosing, screening or assisting screening of whether a subject to be tested suffers from Parkinson's syndrome, wherein the system comprises:
an obtaining device, wherein the obtaining device is used for obtaining the expression level of synaptotagmin-11 in a biological sample; optionally, the biological sample is from the subject to be tested; and
a judging device, wherein the judging device is connected or wirelessly connected to the obtaining device, and the judging device is used for diagnosing, assisting diagnosing, screening or assisting screening of whether a subject to be tested suffers from Parkinson's syndrome based on the expression level of synaptotagmin-11 in biological samples.

Optionally, in the judging device:
the expression level of synaptotagmin-11 in the biological sample is higher than the expression level of synaptotagmin-11 in the normal sample, which is an indication that the corresponding subject to be tested suffers from Parkinson's syndrome; the biological sample is midbrain tissue or body fluid of the subject to be tested; optionally, the body fluid is one or more selected from blood, cerebrospinal fluid and urine.

The fifth purpose of the present application is to provide a biomarker for diagnosing or treating Parkinson's syndrome, wherein the biomarker is Synaptotagmin-11.

Optionally, the synaptotagmin-11 is the synaptotagmin-11 in midbrain tissue or body fluid of the subject to be tested;
optionally, the body fluid is one or more selected from blood, cerebrospinal fluid and urine.

The sixth purpose of the present application is to provide an animal model of Parkinson's syndrome, wherein the expression level of synaptotagmin-11 of the animal model of Parkinson's syndrome is increased compared with wild-type animals.

Optionally, the animal is selected from at least one of mice, rats or primates.

The seventh purpose of the present application is to provide a method for screening a medicant for treating Parkinson's syndrome, wherein the method comprises:
administering a candidate medicant to the animal model;
screening for a candidate medicant that reduces the expression level of synaptotagmin-11, or screening for a candidate medicant that relieves Parkinson's syndrome, and the candidate medicant obtained by the screening being used as the medicant for treating Parkinson's syndrome.

The eighth purpose of the present application is to provide use of a substance for knocking out, silencing or mutating synaptotagmin-11 or reduces the activity of synaptotagmin-11 in preparing a clinical medicant for Parkinson's syndrome, wherein the medicant has a function of any one of the following:
1) reducing the expression level of synaptotagmin-11;
2) reducing or blocking the physiological function of synaptotagmin-11; and
3) being used for treating Parkinson's syndrome.

Optionally, a method for reducing the expression level of the synaptotagmin-11 is any one or more of the following
1) reducing the transcription level of the synaptotagmin-11 coding gene;
2) reducing the translation level of synaptotagmin-11 protein;
3) increasing the synaptotagmin-11 degradation speed; and
4) causing a mutation in the synaptotagmin-11 gene.

Optionally, the medicant reducing or blocking the physiological function of the synaptotagmin-11 is specifically that the medicant reduces or blocks the inhibitory effect of the synaptotagmin-11 on dopamine secretion, or increases dopamine secretion by accelerating endocytosis, accelerating vesicle recycling and other ways.

Optionally, the patient with Parkinson's syndrome is a familial hereditary Parkinson's syndrome patient, or a patient with sporadic Parkinson's syndrome.

The ninth object of the present application is to provide a product for treating Parkinson's syndrome, wherein the product comprises a substance for knocking out, silencing or mutating synaptotagmin-11 or reduces the activity of synaptotagmin-11.

Optionally, the synaptotagmin-11 is the synaptotagmin-11 in midbrain tissue or body fluid of the subject to be tested; optionally, the body fluid is one or more selected from blood, cerebrospinal fluid and urine.

Optionally, the blood is isolated or in vivo blood; midbrain tissue of the subject to be tested is isolated or in vivo midbrain tissue of the subject to be tested.

Optionally, the subjects to be tested are patients with Parkinson's syndrome.

The technical solution of the present application has the following advantages:
Through research, the inventor unexpectedly found that synaptotagmin-11 (Syt11) in peripheral blood can be used as an ideal single diagnostic marker for PD patients. It has been disclosed in the prior art that Syt11 is an important brake protein of endocytosis (EMBO Rep 2016, 17(1):47-63), and it is further disclosed that Syt11 can be used as a substrate of parkin to mediate the pathological process of parkin-related PD (Nat Commun 2018, 9:81). As we all know, parkin mutation is considered to be closely related to familial PD, and the proportion of PD caused by it is very low (<2%, so it is speculated that parkin and its substrates cannot be used as broad-spectrum diagnostic markers of PD). Unexpectedly, in the present application, it is found that the expression level of Syt11 in peripheral blood of sporadic PD patients and PD patients with familial hereditary history were significantly up-regulation, and the expression level of Syt11 in peripheral blood of more than 50% PD patients was highly up-regulation (more than 1.75 times the average of the control group, above the 95% quantile line of the control group), which was much higher than the sum (5% to 10%) of the proportion of all known hereditary PD patients, and more than 93% of the syt11 high expression population were PD patients. More importantly, the inventors have found in MPTP, 6-OHDA, rotenone and other mice PD models that the up-regulation of Syt11 expression starts at the earliest stage of PD (it takes 2 to 3 weeks for the model to be successfully established, and the up-regulation of Syt11 expression starts at the earliest stage of PD). Modeling begins to appear within 12 hours to 24 hours). These breakthroughs show that Syt11 is the only single target marker for PD clinical diagnosis, especially early diagnosis/screening, among the hundreds of PD genetic related genes known so far, and it is also the most ideal PD clinical detection marker so far.

The inventor's early research results showed that the absence of parkin would lead to the up-regulation of Syt11 expression in dopamine neurons in the midbrain, which is the key to mediate the pathological process of parkin-related PD. However, changes in Syt11 expression were not detected in neurons in the hippocampus, another important brain region of the brain. Based on these findings, those skilled in the art generally believe that the up-regulation of Syt11 expression is tissue-specific, and Syt11 in peripheral blood should not reflect the pathological changes of brain tissue of PD patients. At the same time, it is known that parkin has more than a dozen different substrates, and the inactivation of parkin leads to the up-regulation of the expression of these substrates in DA neurons, but none of the known substrates can be used as a peripheral blood marker for independent detection of PD. Further, our earlier studies showed that parkin regulates Syt11 protein level by mediating Syt11 proteasome-dependent degradation process, but Syt11 mRNA and protein levels were significantly up-regulation in PD patients and PD mice models, indicating that there is an important parkin-independent regulatory mechanism for Syt11, which may be a new mechanism leading to the high expression of Syt11 in PD patients/animal models. Therefore, the up-regulation of Syt11 mRNA and protein expression in the peripheral blood of PD patients is an unexpected finding. What is even more unexpected is that Syt11 is not only the only single peripheral blood marker among pakrin substrates that can be used for clinical diagnosis of PD, but its high expression ratio (>50%) far exceeds the ratio of parkin itself in PD (<2 %, Neurology 2001, 57: 359-362; Parkinsonism and Related Disorders 2012, 18: S66-70), even far exceeding the sum of the proportion of all hereditary PD in all PD cases (<10%), and its up-regulation has appeared as early as the early stage of PD. Therefore, using Syt11 as a marker for early screening of hereditary and sporadic PD patients, regardless of its scope of application (early screening, clinical diagnosis, typing analysis and routine physical examination of older people for more than 50% of PD patients), or its detection efficacy (the positive correct rate is more than 93%, 5 to 10 years earlier than the clinical diagnosis), all have achieved unexpected results.

In conclusion, the early diagnosis of PD is a major worldwide scientific and medical problem that has long been hoped to be solved in this field but has never been solved. The inventors unexpectedly found that the high expression of Syt11 in midbrain tissue and peripheral blood is an important marker of sporadic and familial hereditary PD, the Syt11 gene and its protein expression level are by far the most ideal clinical diagnosis of PD, especially an important basis for early clinical diagnosis.

### BRIEF DESCRIPTION OF THE DRAWINGS

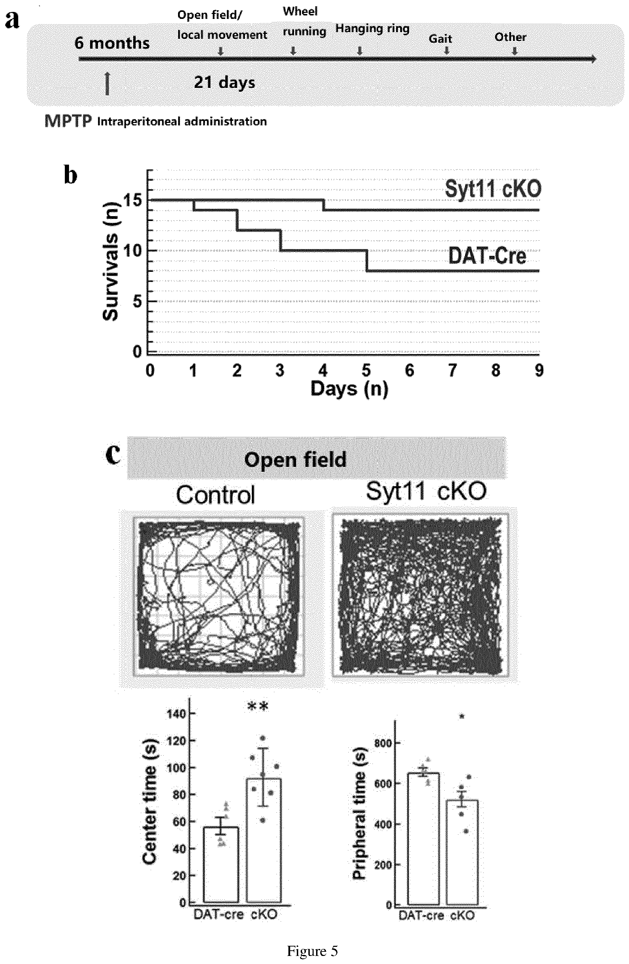

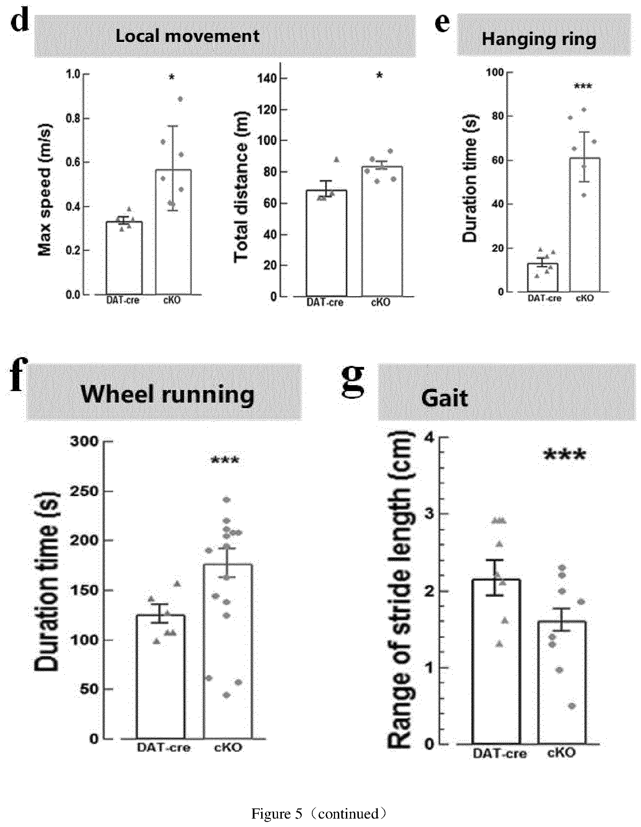

In order to illustrate the technical solutions in the specific embodiments of the present application or in the prior art more clearly, the drawings to be used in the description of the specific embodiments or the prior art will be briefly introduced below. Obviously, the drawings in the following description show some of the embodiments of the present application, and those of ordinary skill in the art may still obtain other drawings from these drawings without creative efforts.
Figure 1 is the expression level of Syt11 in the peripheral blood of PD patient and control population among the Example 1; wherein Figure 1a shows some representative Western blot bands of Syt11 expression levels in peripheral blood of control (Ctrl) and random PD patients, and Figure 1b shows the statistical analysis of Syt11 expression levels in peripheral blood of control (Ctrl, n=45) and PD patients (n=53);
Figure 2 is the statistical analysis of the up-regulation of Syt11 protein expression in MPTP-induced PD mice model brain tissue in Example 2;
wherein Figure 2a is a schematic diagram of the main projection areas (striatum, prefrontal cortex, etc.) of midbrain dopamine neurons (SNc, VTA);
Figure 2b uses the open field to evaluate the athletic ability of mice, and verifies the success of PD modeling in MPTP mice (Saline is the saline control group); in Figure 2b, the ordinates are duration time (duration time of movement), max speed (maximum speed of movement) and distance (movement distance), respectively;
Figure 2c and Figure 2d are the statistical analysis of the expression levels of Syt11 protein in the striatum and substantia nigra after MPTP injection at different times (12 hours, 1 day, 5 days, 9 days and 21 days); in Figure 2c and Figure 2d, the ordinate is Syt11 (fold to Ctrl);
Figure 3 is the expression level of Syt11 protein in hippocampal neurons of MPTP-induced PD mice (n= 7) and control mice (n= 8) in Example 3;
Figure 4 is the statistical analysis of the Syt11 expression level in the blood of MPTP-induced PD mice model in Example 4; the left figure of Figure 4 shows the variation of Syt11 protein expression level in PD mice (n=7) and control mice (n=6) after 5 days of MPTP induction; the right figure of Figure 4 shows the variation of Syt11 mRNA transcript levels in peripheral blood after MPTP injection for different times (n=7 for each condition, each group);
Figure 5 shows the results of Example 5 in which specific knockout of Syt11 in DA neurons significantly reversed the pathological process of PD;
wherein Figure 5a is the experimental flow chart of Example 5; Figure 5b is the statistical analysis of the mortality of Syt11 knockout mice (cKO) and control mice (DAT-Cre) during and after MPTP injection, and the mortality of Syt11 cKO mice is greatly reduced; Figure 5c is the time of the mice in the center and edge of the open field in the anxiety-like behavior of the open field test; and Figure 5d is the maximum movement speed and movement distance of the mice after MPTP treatment in the open field test;
Figure 5e is the duration time of mice on the copper wire after MPTP treatment in the wire hanging (ring hanging) test;
Figure 5f is the duration time of mice on the wheel after MPTP treatment in the wheel running test;
Figure 5g is the overlap of the front and rear paws of the mice after MPTP treatment in the gait experiment; the range of stride length is the stride range;

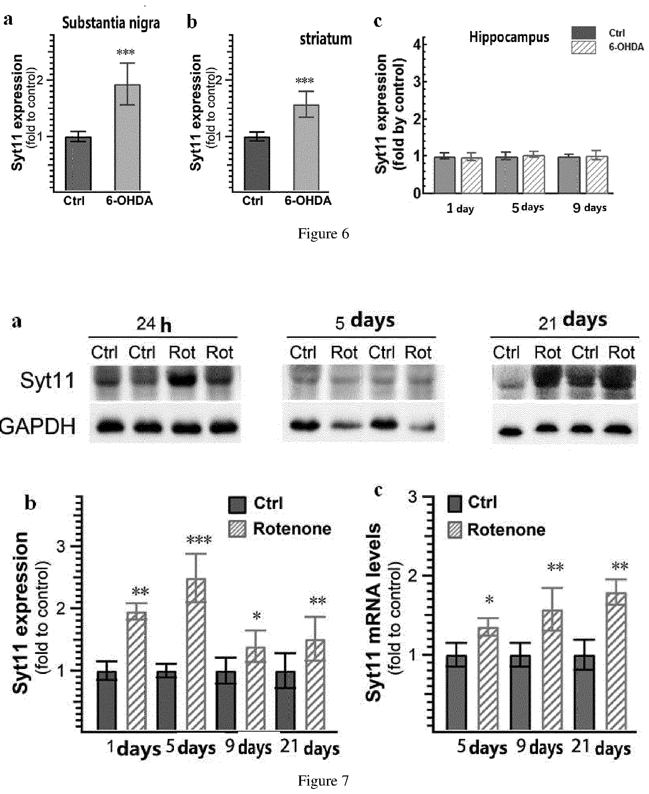

Figure 6 is a statistical analysis of the expression level of Syt11 in the 6-OHDA-induced PD mice model in Example 6;
wherein Figure 6a and Figure 6b show the expression levels of Syt11 protein in the substantia nigra (SNc) and striatum of 6-OHDA-induced PD mice (n=7) and control mice (n=8);
Figure 6c is the expression level of Syt11 protein in the hippocampus (Hippo) (each condition, each group, n=7);
Figure 7 is the statistical analysis of Syt11 expression level in blood of rotenone-induced PD mice model in Example 7;
wherein Figure 7a shows some representative Western blot bands of Syt11 expression levels in peripheral blood of rotenone-induced PD mice (Rot) and control mice (Ctrl);
Figure 7b is a statistical diagram of the expression level of Syt11 protein in peripheral blood after rotenone injection at different times (each condition, each group, n=6);
Figure 7c shows the variation of Syt11 mRNA transcription levels in the peripheral blood of PD mice (Rotenone, n=5) and control mice (Ctrl, n=4) after rotenone injection at different times;

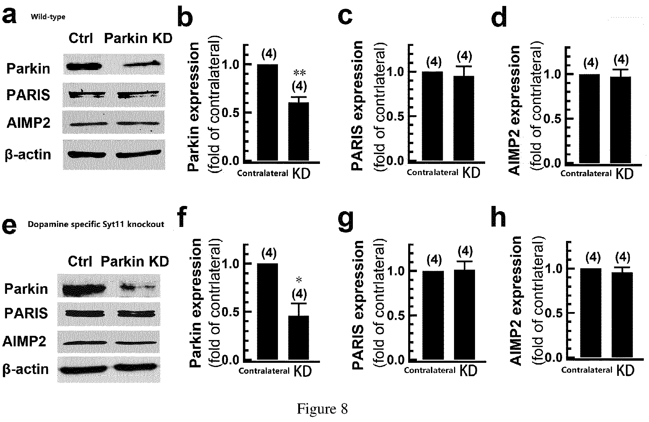

Figure 8 shows the effect of parkin knockdown on the expression levels of its substrates PARIS and AIMP2 in the mice model in Example 8;
wherein Figure 8a shows the expression levels of Parkin, PARIS and AIMP2 on the left and right sides of wild-type (WT) mice after unilateral parkin knockout;
Figure 8b to Figure 8d are the statistical graphs of the expression levels of Parkin, PARIS and AIMP2 respectively, Contra is the control side without virus injection, and KD is the injection side (n=4);
Figure 8e to Figure 8h show the effects of unilateral parkin knockout on the expression levels of PARIS and AIMP2 in Syt11 knockout rats (cKO), respectively, and KD is the injection side (n=4).

### DETAILED DESCRIPTION

The following examples are provided for a better understanding of the present invention and do not constitute a limitation on the content or scope of protection of the present invention.

Unless otherwise specified, the reagents and culture media used in the following examples are all commercially available, and the experimental methods without specific conditions are usually in accordance with conventional conditions, and the nucleic acid electrophoresis, Western blot, real time PCR and other operations used are all proceed as usual.

DAT-Cre mice, Syt11 flox mice, and shRNA lentiviruses were disclosed in Wang C, Kang X, Zhou L, et al. Synaptotagmin-11 is a critical mediator of parkin-linked neurotoxicity and Parkinson's disease-like pathology[J].NATURE COMMUNICATIONS, 2018, 9(1):81.

IP cell lysate: containing 2% (volume ratio) of cocktail (539134, Calbiochem) and 1% (volume ratio) of PMSF.

### Example 1. The expression level of Syt11 in the peripheral blood of Parkinson's patients was significantly increased

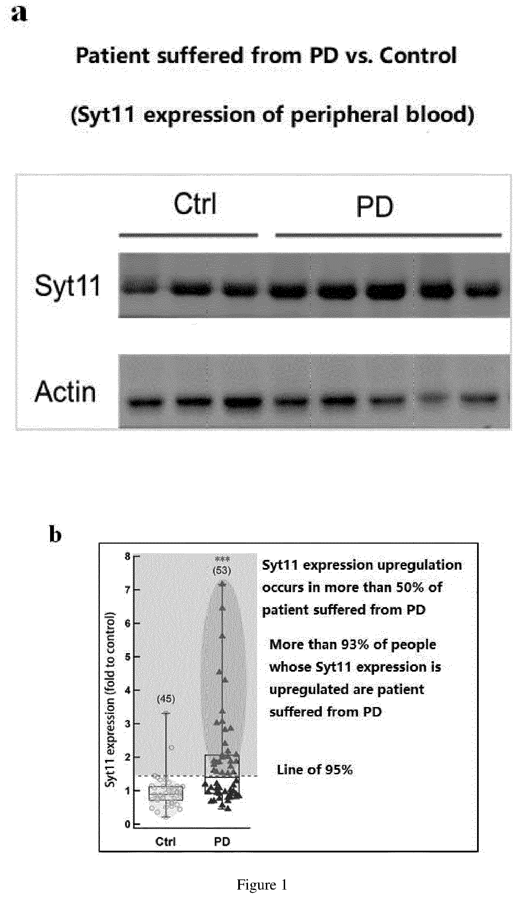

1. Extraction of Syt11 protein from peripheral blood
   5 µL human blood sample (10 µL mice blood and 90 µL IP) was taken and added to 95 µL IP cell lysate, after homogenization, centrifuged at 12000g at 4°C for 15 minutes. The supernatant was taken, and 5 × SDS-PAGE loading buffer with 1/4 supernatant volume was added, and boiled at 95°C to 100°C for 10 min to obtain protein samples for Western blot analysis.
2. Western blot: The obtained protein solution was subjected to electrophoresis and transferred to a PVDF membrane. The TBST solution containing 5% defatted milk powder was sealed at room temperature for 1 hour to 1.5 hours, washed three times, and incubated overnight at 4°C in a 2% BSATBST solution containing Syt11 primary antibody (SYSY, 270 003). The corresponding secondary antibodies (111-035-003 rabbit antibody, 115-035-003 mice antibody, Jackson ImmunoResearch) were incubated at room temperature for 1.5 hours to 2 hours after washing the membrane five times with TBST, the membrane was scanned on the clinx chemicapture imaging system after washing five times with TBST. The results were shown in Figure 1, the expression level of Syt11 protein in the peripheral blood of PD patients was significantly up-regulation than that of the control group (normal people) (***p<0.001); the PD patients in Figure 1 were randomly selected PD patients, a total of 53 cases (27 cases showed significant up-regulation of Syt11), of which 6 cases had familial genetic history (4 cases showed up-regulation of Syt11 expression), 47 cases were sporadic Parkinson's disease patients (23 cases showed up-regulation of Syt11 expression). The peripheral blood of PD patients is the peripheral blood of clinically diagnosed Parkinson's syndrome patients, provided by the First Affiliated Hospital of Xi'an Jiaotong University, Dalian People's Hospital, and Xingtai People's Hospital, and the patients were informed. 45 normal subjects were from the routine physical examination population without Parkinson's disease. Taking the upper 95% of the control group (Ctrl) as the quantile line, more than 50% of PD patients have significantly up-regulation Syt11 protein expression, which is much higher than the sum of all other genetic factors (5% to 10%), and more than 93% of the population with up-regulation expression of Syt11 are PD patients, indicating that Syt11 is the most ideal peripheral blood PD clinical detection marker among hundreds of PD genetic related genes known so far.

### Example 2. The expression of dopamine neuron Syt11 in MPTP-induced PD mice model is significantly up-regulated

### 1. Modeling of PD mice

MPTP was injected intraperitoneally at 25 mg/kg/day for 5 consecutive days to establish mice PD models.

### 2. Open field experiment

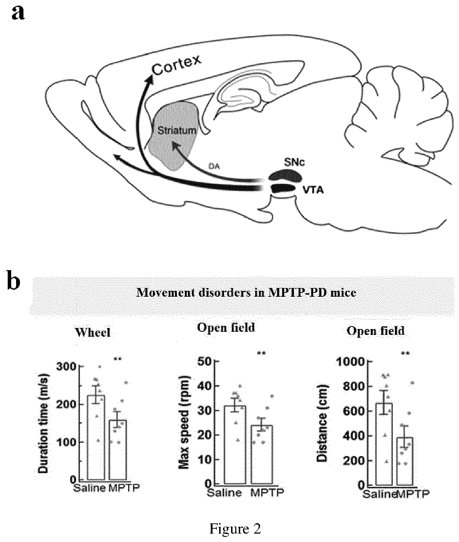

The experimental animals were gently taken out of the breeding cage, and quickly placed in the central area of the open field experimental device (50cm×50cm×40cm), and the movements of the animals in the open field were automatically recorded by Anymaze analysis software. The experimental time was 30min. The movement distance, average speed, maximum speed, etc. of the experimental animals was automatically counted by the software to indicate the athletic ability of the mice. The results (see Figure 2b) showed that, the MPTP injection group had a significant down-regulated in duration time of movement, maximum movement speed and movement distance compared with the saline injection group, indicating that the PD mice were successfully modeled. At the same time, software was used to automatically count the residence time and entry times of the experimental animals in the central area (20cm×20cm), and the residence time and entry times of the peripheral area (5cm away from the box wall) to indicate the anxiety condition of the mice.

### 3. Brain tissue protein extraction

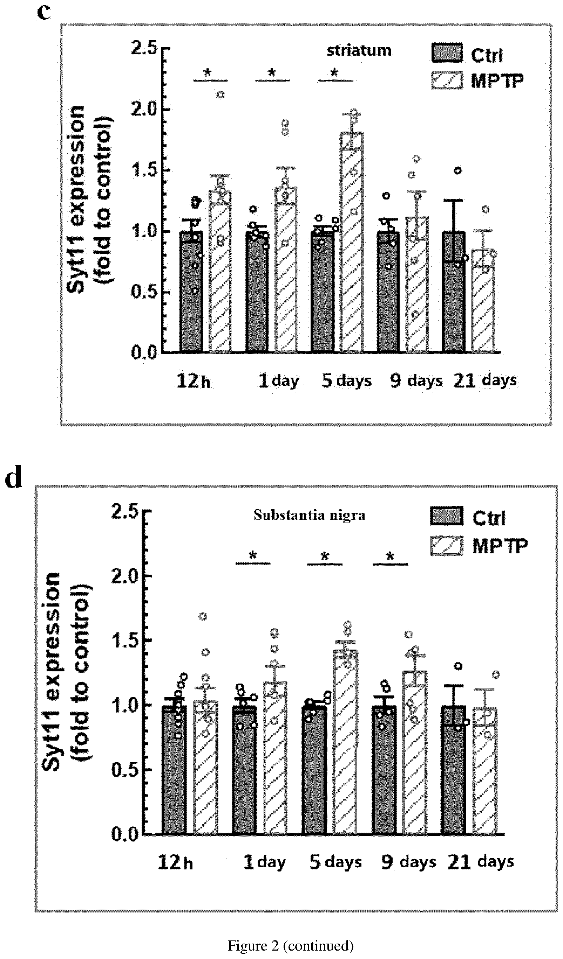

After the mice were anesthetized, they were perfused with 20ml of ice-cold artificial cerebrospinal fluid, and their heads were quickly decapitated to take out the brain tissue, and the hippocampus, striatum, and substantia nigra were cut into horizontal slices of 300 µm thick on a Leica VT1200S microtome. Tissue homogenization was performed, and the homogenized tissue was centrifuged at 12000 g for 15 min at 4°C. The supernatant was taken, , and 5 × SDS-PAGE loading buffer with 1/4 supernatant volume was added, and boiled at 95°C to 100°C for 10 min to obtain protein samples for Western blot analysis. The results showed that the expression level of Syt11 protein in the MPTP injection group was significantly up-regulated in both the soma region of dopamine neurons (substantia nigra, SNc, Figure 2d) and its projection region (striatum, Figure 2c). More importantly, a significant up-regulation in the expression of Syt11 protein was detected in the striatum 12 hours after the first injection of MPTP (p<0.05), and a significant increase in the expression of Syt11 protein was also detected in the substantia nigra 1 day after injection (p<0.05). It takes 2 weeks to establish a PD mice model using MPTP, and the expression of Syt11 has been significantly up-regulation on the day of the first injection of MPTP, indicating that the up-regulation of Syt11 protein expression is a very early event of PD.

### Example 3. The expression level of Syt11 in the hippocampal neurons in MPTP-induced PD mice model has no obvious abnormality

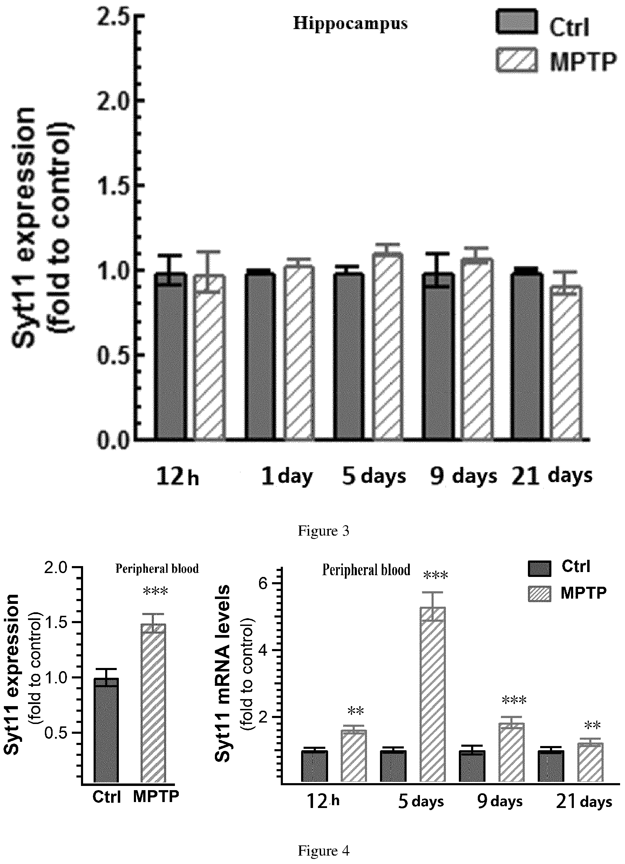

Mice (control group, and MPTP model mice) were anesthetized to separate the hippocampal tissue, total protein was extracted for Western blot analysis, the results were shown in Figure 3, no difference in the expression of Syt11 was detected in the hippocampal tissue until the day 21 of MPTP modeling (from day 1 of the first injection of MPTP), indicating that MPTP can specifically lead to the up-regulation of the expression of Syt11 in the substantia nigra and striatum (brain nuclei associated with PD).

### Example 4. The expression level and transcription level of Syt11 in the hippocampal neurons in MPTP-induced PD mice model has significantly up-regulated

1. The Western blot method for the expression level of Syt11 protein in peripheral blood of mice was shown in Example 1. The results showed that after 5 days of MPTP induction, the expression level of Syt11 protein in the peripheral blood of mice in the PD group was significantly up-regulated (Figure 4a). These results suggest that although the up-regulation of Syt11 expression in brain tissue is specific to brain regions, the expression level of Syt11 in peripheral blood may be an important marker of the pathological process of PD.
2. The transcription level of Syt11 mRNA in mice blood
   (1) Blood lysis: 0.25ml blood sample was transfer to a centrifuge tube, 0.75m1 RNAiso Blood (9112, TAKARA) was added, the mixture was repeatedly pipetted up and down 20 times until the cells were completely lysed.
   (2) RNA extraction: chloroform (0.2 ml) was added to the homogenized lysate, mixed until the solution emulsifies and turns milky white, and stood at room temperature for 5 minutes. It was centrifuged at 12000g at 4°C for 15min, the supernatant was taken, an equal volume of isopropanol was added to the supernatant, the centrifuge tube was inverted up and down to mix well, and stood at 4°C for 30min. It was centrifuged at 12000g, 4°C for 10min, and RNA precipitation appear at the bottom of the test tube. The supernatant was discarded, an equal amount of 75% (v/v) ethanol was added to shake and wash, centrifuged at 7500g at 4°C for 5min, and the supernatant was discarded. The precipitate was dried at room temperature, and an appropriate amount of RNase-free water was added to dissolve the precipitate.
   (3) Reverse transcription: a reverse transcription kit (RR047A, TAKARA) containing Oligo dT Primer was used, RNA template was added, genomic DNA was removed at 42°C for 2min to 5min, then a PCR amplification instrument was used for reverse transcription at 37°C for 15min and at 85°C for 5s.
   (4) Quantification of Syt11: CFX96 Real-time PCR Detection System was used for detection, 10 µL of PCR reaction solution was prepared, 5 µL of TB Green (TAKARA, RR820), 0.4 µL of each Syt11 primer (GenBank number: 229521), 40 ng of cDNA template, and sterilize Water 3.4 µL. The real time PCR amplification results were shown in the right figure of Figure 4. The transcription level of Syt11 mRNA in the blood was significantly up-regulated 12 hours after MPTP injection (p<0.01) compared with the normal saline group, and the transcription level up-regulated more significantly (p<0.001) after 5 days and this up-regulation lasted for at least 21 days. These results indicated that the transcription level and expression level of Syt11 in peripheral blood both start at the early stage of PD pathological process, and can be used as an ideal marker for early clinical diagnosis of PD.

### Example 5. Syt11 knockout reverses behavioral variation of MPTP-induced PD

### 1. Syt11 knockout improved the survival rate of MPTP-PD mice

DAT-Cre mice were mated with Syt11 flox mice to generate dopamine neuron-specific Syt11 knockout mice (Syt11 cKO). Six-month-old Syt11 cKO mice and DAT-Cre control mice were used as experimental subjects, and MPTP (25 mg/kg) was injected intraperitoneally for 5 consecutive days to establish a PD mice model. The results showed that the mortality of Sytll cKO rats was significantly reduced after 5 consecutive days of MPTP process (Figure 5a to Figure 5b) compared with the DAT-Cre control rats, indicating that the knockout of Syt11 can reduce the toxicity of MPTP and protect the survival of mice.

### 2. Syt11 knockout relieved anxiety-like behavior in MPTP-PD mice

Anxiety-like behaviors were tested by the open field test as described in Example 2. The results were shown in Figure 5c. The results showed that the control mice did have PD-like anxiety-like symptoms after MPTP injection, and the exploration time of the Syt11-cKO mice to the center of the open field was significantly higher than that of the control mice, and the time at the edge of the open field significantly shortened (Figure 5c), indicating that Syt11 knockout can alleviate the anxiety-like behavior of MPTP-PD mice.

### 3. Syt11 knockout reversed the movement behavior of MPTP-PD mice

(1) Open field experiment was performed as described in Example 2. The results were shown in Figure 5d. The Syt11 cKO mice showed a significant increase in both max speed and total distance compared with the DAT-Cre control mice (p<0.05).
(2) Wire hanging experiment: a tight copper wire was hanged at 60 cm above a large rearing box filled with litter. Mice were gently placed on the copper wire and held in an upside-down posture. When the mice were not strong enough, they choose to jump into the breeding box, and the time spent on the copper wire can be recorded to reflect the athletic ability of the mice. The results are shown in Figure 5e. The duration time of Syt11 cKO mice in the copper wire was significantly increased (p<0.001) compared with DAT-Cre control mice.
(3) Wheel running test: the Ugo Basile was set at an initial speed of 5 rpm, and accelerated after 5 minutes until the final speed was 40 rpm. Hold the mice by its tail, place it on the Ugo Basile, with its back to the observer, and make sure the mice do not turn. Acceleration was started after the mice had adapted for 30 s, and the time and rotational speed of the mice falling from the Ugo Basile were recorded. In this test, through 4 days of training, three consecutive training sessions per day, the formal experiment was carried out on the 5 day, and the falling time and rotational speed were recorded (the maximum value is 5 minutes if it does not fall for more than 5 minutes). The results are shown in Figure Sf, the duration time of Syt11 cKO mice on the wheel was significantly increased (p<0.001) compared with DAT-Cre mice.
(4) Gait analysis: two kinds of non-toxic pigments, red and black, were painted on the front and rear soles of the mice respectively. The experimental mice were trained to run three times every day on a track with a length of 100 cm, a width of 10 cm and a height of 10 cm. Carry out gait experiment and analysis, count step distance and the overlapping situation (palm distance) of front and back soles, to evaluate the stability of mice's steps. The results are shown in Figure 5g. The differential fluctuation of the front and rear paws of the Syt11 cKO mice was significantly reduced, and the overlap of the front and rear paws was increased compared with the DAT-Cre control mice.

The above results indicated that the up-regulation of Syt11 expression in dopamine neurons and peripheral blood is one of the important pathogenic mechanisms in the early stage of Parkinson's disease. Up-regulation of Syt11 expression can not only serve as an important early diagnostic marker for PD, but targeting Syt11 can also fully reverse PD-related movement symptoms and non- movement symptoms, which is an effective medicant target for PD treatment.

### Example 6. The expression level of Syt11 in the hippocampal neurons in 6-OHDA (6-hydroxydopamine)-induced PD mice model has significantly up-regulated

### 1. Stereotaxic injection of 6-OHDA into the brain to establish a unilateral PD mice model.

Mice were anesthetized by intraperitoneal injection of urethane at 1.5 g/kg, and their body temperature was maintained at 37°C with a heating blanket, while an oxygen mask was used to provide oxygen to keep the mice in good condition. The mice were fixed on the brain stereotaxic instrument, and the anterior and posterior fontanelle were kept on the same plane. The coordinates (AP: -2.1mm, ML:1.1mm) was used to find the plane position of the medial forebrain bundle (MFB), a skull drill was used to drill a hole, and a syringe with a 32G needle was inserted into the brain at -4.3mm on the plane of the skull. The injection dose of 6-OHDA was 2.5µg/mice.

### 2. Western blot analysis

Western blot analysis was performed as described in Example 1. The results showed that, the expression of Syt11 in the 6-OHDA injection side was significantly up-regulated in both the substantia nigra (Figure 6a) and the striatum (Figure 6b) (p <0.001) compared with the control side without 6-OHDA injection, but no significant difference was found on both sides of the hippocampus (Figure 6c), indicating that the up-regulation of Syt11 expression is also applicable to the PD mice model made by 6-OHDA, suggesting that the up-regulation of Syt11 expression is a common pathological mechanism of sporadic PD.

### Example 7. The expression level of Syt11 in the hippocampal neurons in rotenone-induced PD mice model has significantly up-regulated

30 mg/kg rotenone was administered by intragastric administration for 21 consecutive days to establish a PD mice model. 10 µL mice blood sample was taken and 90 µL IP cell lysate was added to extract total protein for Western blot analysis. The results were shown in Figure 7a to Figure 7b, the expression of Syt11 in peripheral blood can be detected up-regulated on the day of intragastric administration of rotenone, and this up-regulated level can last until the PD mice model is established. At the same time, the transcription level of Syt11 mRNA also showed a similar up-regulation trend, further proving that the up-regulation of Syt11 expression is a common pathological mechanism of PD, and peripheral blood Syt11 mRNA and Syt11 protein are ideal markers for early screening and clinical diagnosis of PD.

### Example 8. The expression levels of PARIS and AIMP2, the important substrates of Parkin, in the PD mice model were not significantly abnormal

The inventor's previous work (prior art) showed that Syt11 was a substrate of parkin, but there are many substrates of parkin. However, most of the substrates cannot be proved to mediate the pathological process of PD. Therefore, in addition to Syt11, this Example further tested whether the other two substrates of parkin, PARIS and AIMP2, are also up-regulated in the pathological process of PD.

### 1. Parkin shRNA virus injection

Mice were anesthetized by intraperitoneal injection of urethane at 1.5 g/kg, and their body temperature was maintained at 37°C with a heating blanket, while an oxygen mask was used to provide oxygen to keep the mice in good condition. The mice were fixed on the brain stereotaxic instrument, and the anterior and posterior bregma were kept on the same plane. The coordinates (AP: -3mm, ML: 1.25mm) was used to find the plane position of the substantia nigra, a skull drill was used to drill a hole, a syringe with a 32G needle was inserted into the brain 4mm on the plane of the skull, and 1 µl of parkin shRNA lentivirus concentrate (titer: 10⁸-10⁹/ml) was injected slowly at a speed of 100nl/min, stayed for 15 to 20 minutes after the injection, the syringe was pulled out slowly, and the mice was placed on a 37°C heating blanket to recover after the scalp was sutured.

### 2. Western blot

The results were shown in Figure 8. This example found that after unilateral parkin knockdown (KD) in wild-type (WT) mice, the expression level of parkin was significantly reduced, but there was no difference in the expression of PARIS and AIMP2 on both sides (Figure 8ato Figure 8d), showing that parkin knockdown did not affect the expression of PARIS and AIMP2 in the PD model. In order to further detect whether Syt11 has an effect on the expression of these two substrates, this example further repeated the experiment on Syt11 cKO mice, and found that after unilateral parkin knockdown, the expression levels of PARIS and AIMP2 still did not appear in the bilateral brain regions The difference (Figure 8e- Figure 8h), indicating that the expression of Syt11 in the parkin knockdown PD model also had no effect on the expression of PARIS and AIMP2. These results indicated that, unlike other parkin substrates, Syt11 was the only known single marker that can be used for early screening and diagnosis of PD.

Obviously, the above examples are merely examples made for clear description, rather limiting the implementations. For those of ordinary skill in the art, other different forms of variations or modifications can also be made on the basis of the above-mentioned description. All embodiments are not necessary to be and cannot be exhaustively listed herein. In addition, obvious variations or modifications derived therefrom all fall within the scope of protection of the present invention.

## Claims

1. Any one of the following use:
1) use of a substance for measuring the content of synaptotagmin-11 in preparing a product for diagnosing or assisting diagnosing of whether a subject to be tested suffers from Parkinson's syndrome;
2) use of a substance for measuring the content of synaptotagmin-11 in preparing a product for screening or assisting screening of whether a subject to be tested suffers from Parkinson's syndrome;
3) use of a substance for measuring the content of synaptotagmin-11 in diagnosing or assisting diagnosing of whether a subject to be tested suffers from Parkinson's syndrome; and
4) use of a substance for measuring the content of synaptotagmin-11 in screening or assisting screening of whether a subject to be tested suffers from Parkinson's syndrome.

2. Any one of the following use:
1) use of synaptotagmin-11 as a marker of Parkinson's syndrome in diagnosing or assisting diagnosing of whether a subject to be tested suffers from Parkinson's syndrome; and
2) use of synaptotagmin-11 as a marker in screening or assisting screening of whether a subject to be tested suffers from Parkinson's syndrome.

3. A product for diagnosing or assisting diagnosing of whether a subject to be tested suffers from Parkinson's syndrome, wherein the product diagnoses, assisting diagnoses, screens or assisting screens whether a subject to be tested suffers from Parkinson's syndrome based on the expression level of synaptotagmin-11 in biological samples;
optionally, the product is a kit; optionally, the kit comprises reagents for specifically detecting the expression level of synaptotagmin-11;
optionally, the reagents comprise an antibody, a protein and/or a polypeptide that specifically bind to the synaptotagmin-11 protein.

4. The product of claim 3, wherein the antibody, the protein and/or the polypeptide is modified with isotopes, horseradish peroxidase, colloidal gold, fluorescent probes, etc., or is not modified.

5. A system for diagnosing, assisting diagnosing, screening or assisting screening of whether a subject to be tested suffers from Parkinson's syndrome, wherein the system comprises:
an obtaining device, wherein the obtaining device is used for obtaining the expression level of synaptotagmin-11 in a biological sample; and
a judging device, wherein the judging device is connected to or wirelessly connected the obtaining device, and the judging device is used for diagnosing, assisting diagnosing, screening or assisting screening of whether a subject to be tested suffers from Parkinson's syndrome based on the expression level of synaptotagmin-11 in biological samples.

6. The system of claim 5, wherein in the judging device:
the expression level of synaptotagmin-11 in the biological sample is higher than the expression level of synaptotagmin-11 in the normal sample, which is an indication that the corresponding subject to be tested suffers from Parkinson's syndrome; the biological sample is midbrain tissue or body fluid of the subject to be tested; optionally, the body fluid is one or more selected from blood, cerebrospinal fluid and urine.

7. A biomarker for diagnosing or treating Parkinson's syndrome, wherein the marker is Synaptotagmin-11;
optionally, the synaptotagmin-11 is the synaptotagmin-11 in midbrain tissue or body fluid of the subject to be tested;
optionally, the body fluid is one or more selected from blood, cerebrospinal fluid and urine.

8. An animal model of Parkinson's syndrome, wherein the expression level of synaptotagmin-11 in the midbrain tissue or body fluid of the animal model of Parkinson's syndrome is increased compared with wild-type animals.

9. The animal model of Parkinson's syndrome of claim 8, wherein the animal is selected from at least one of mice, rats or primates.

10. A method for screening a medicant for treating Parkinson's syndrome, wherein the method comprises:
administering a candidate medicant to the animal model of claim 8 or 9;
screening for a candidate medicant that reduces the expression level of synaptotagmin-11 or reduces the activity of synaptotagmin-11, or screening for a candidate medicant that relieves Parkinson's syndrome, and the candidate medicant obtained by the screening being used as the medicant for treating Parkinson's syndrome.

11. Use of a substance for knocking out, silencing or mutating synaptotagmin-11 or reduces the activity of synaptotagmin-11 in preparing a clinical medicant for Parkinson's syndrome, the medicant has a function of any one of the following:
1) reducing the transcription level or expression level of synaptotagmin-11;
2) reducing or blocking the physiological function of synaptotagmin-11; and
3) being used for treating Parkinson's syndrome.

12. The use of claim 11, wherein a method for reducing the expression level of the synaptotagmin-11 is any one or more of the following
1) reducing the transcription level of the synaptotagmin-11 coding gene;
2) reducing the translation level of synaptotagmin-11 protein;
3) increasing the synaptotagmin-11 degradation speed; and
4) causing a mutation in the synaptotagmin-11 gene;
optionally, the medicant reducing or blocking the physiological function of the synaptotagmin-11 is specifically that the medicant reduces or blocks the inhibitory effect of the synaptotagmin-11 on dopamine secretion, or increases dopamine secretion by accelerating endocytosis, accelerating vesicle recycling and other ways.

13. The use of claim 11 or 12, wherein the patient has a familial genetic history of Parkinson's syndrome, or the patient is a sporadic Parkinson's syndrome patient.

14. A product for treating Parkinson's syndrome, wherein the product comprises a substance for knocking out, silencing or mutating synaptotagmin-11 or reduces the activity of synaptotagmin-11.

15. The use of claim 1 or 2, the product of claim 3 or 4, the marker of claim 7, the animal model of claim 8 or 9, or the method of claim 10, the use of any one of claims 11-13, wherein the synaptotagmin-11 is the synaptotagmin-11 in the brain tissue or body fluid of a human or an animal model of Parkinson's disease;
optionally, the body fluid is selected from one or more of blood, cerebrospinal fluid and urine;
optionally, the subjects to be tested in claims 1 and 2 are patients with Parkinson's syndrome, suspected patients with Parkinson's syndrome, high-risk groups of Parkinson's syndrome or immediate relatives of patients with Parkinson's syndrome; patients with Parkinson's syndrome is a sporadic Parkinson's syndrome patient or a familial hereditary Parkinson's syndrome patient; the biological sample in claims 3 and 5 is from a subject to be tested.
